Europäisches Patentamt

European Patent Office

Office européen des brevets

(11) Publication number: **0 174 011**
**B1**

# EUROPEAN PATENT SPECIFICATION

(45) Date of publication of patent specification: **13.06.90**

(21) Application number: **85111125.2**

(22) Date of filing: **03.09.85**

(51) Int. Cl.⁵: **A 61 M 5/158,** A 61 M 1/02,
C 08 K 7/14

(54) **Medical puncture needle.**

(30) Priority: **05.09.84 JP 184402/84**

(43) Date of publication of application:
**12.03.86 Bulletin 86/11**

(45) Publication of the grant of the patent:
**13.06.90 Bulletin 90/24**

(84) Designated Contracting States:
**BE DE FR GB IT**

(56) References cited:
FR-A-2 107 538
US-A-4 411 661

(73) Proprietor: **TERUMO KABUSHIKI KAISHA
trading as TERUMO CORPORATION
44-1, 2-chome, Hatagaya Shibuya-Ku
Tokyo 151 (JP)**

(72) Inventor: **Hirokawa, Hiroshi
894-1, Miyabara-cho
Koufu-shi Yamanashi-ken (JP)**
Inventor: **Nakada, Tsuneo
1361, Ikkui Showamachi
Nakakoma-gun Yamanashi-ken (JP)**

(74) Representative: **Henkel, Feiler, Hänzel & Partner
Möhlstrasse 37
D-8000 München 80 (DE)**

Courier Press, Leamington Spa, England.

## Description

Background of the invention
Field of the invention

This invention relates to a medical puncture needle. More particularly, this invention relates to a medical puncture needle made of fiber-reinforced thermoplastic resin used in medical tools such as fluid transfusion set, blood transfusion set, and connecting tube set.

Description of prior art

Heretofore, in the use of a fluid transfusion set or a blood transfusion set, necessary release of fluid from the fluid container or blood from the blood container is effected by causing a vial needle joined to a transfusion tube to pierce a rubber plug or resin diaphragm fitted in the fluid or blood container. The conventional vial needle has been formed of metal such as stainless steel or acrylonitrile-butadiene-styrene (ABS) resin. There is also known (US—A—4411661) a spike connector molded of polypropylene in an integral, one-piece construction.

When the vial needle is made of metal, there ensue the disadvantages that the pointed edge of the needle will possibly inflict a cut on the user's finger or, when the needle pierces the rubber plug, a bit cored from the rubber plug will enter the fluid, the needle itself will gather rust or will defy integration with the hub part made of resin, and the process of production will be consequently complicated. On the other hand, when the vial needle is made of resin, there are the disadvantages that the needle, while in the process of piercing the rubber plug, will encounter strong resistance, with the magnitude of resistance gradually increased in proportion to the number of repetitions of piercing, and the needle will break owing to shortage of strength.

An object of this invention, therefore, is to provide a novel medical puncture needle.

Another object of this invention is to provide a medical puncture needle which proves safe on the part of the user, minimally entails the phenomenon of coring, avoids gathering rust, encounters insignificant resistance while piercing the rubber plug, and enjoys improved resistance to breakage.

Summary of the invention

The objects described above are accomplished by a medical puncture needle formed of a hardened mixture comprising a thermoplastic resin and a fibrous material dispersed in said resin and endowed with hardness exceeding 100 on the Rockwell hardness scale R and enabled to pierce an elastic or flexible object.

This invention also resides in a puncture needle having the fibrous material incorporated in an amount of 10 to 90 parts by weight based on 100 parts by weight of the thermoplastic resin. This invention further resides in a puncture needle having glass fibers or carbon fibers as the fibrous material. Further, this invention resides in a puncture needle having the individual fibers of the fibrous material in diameters not exceeding 25 µm and in lengths not exceeding 10 mm. This invention is directed to a puncture needle having the thermoplastic resin selected from the group consisting of polyolefins, ABS resin, and thermoplastic polyester resins. This invention is also directed to a puncture needle having a polyolefin, specifically polypropylene, as the thermoplastic resin. This invention is further directed to a puncture needle having a thermoplastic polyester resin, specifically polyethylene terephthalate, as the thermoplastic resin. Further this invention is directed to a puncture needle having a resin capable of resisting gamma rays as the thermoplastic resin. This invention pertains to a puncture needle having the individual fibers of the fibrous material coated with an agent for conferring on the fibers affinity for the resin. This invention further pertains to a puncture needle so molded as to acquire hardness exceeding 110 on the Rockwell hardness R scale. This invention also pertains to a puncture needle having a hollow construction throughout the entire length thereof except for the piercing tip which has a substantially curved surface not more than 1.0 mm in radius of curvature.

Brief description of the drawings

Figs. 1 and 2 are side views of a typical puncture needle as one embodiment of this invention,
Fig. 3 is a side view of another typical puncture needle as another embodiment of this invention, and
Figs. 4 through 6 are sectional views of typical rubber plugs to be pierced by the puncture needle of this invention.

Description of preferred embodiment

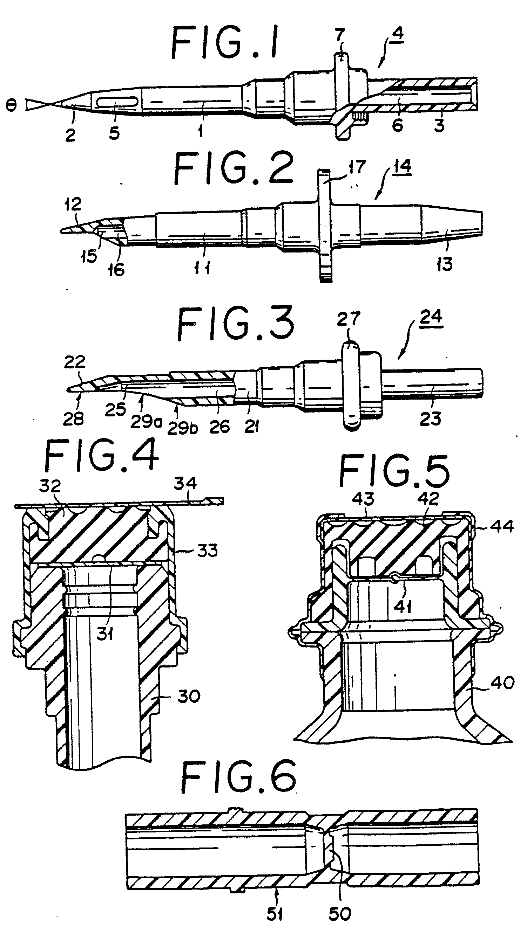

Now, one embodiment of this invention will be described with reference to the drawings. As illustrated in Fig. 1, a vial needle 4 comprises a main body portion 1, a piercing tip portion 2 formed at one end of the main body portion 1, and a connection portion 3 formed at the other end of the main body portion 2 and adapted to be joined with a fluid transfusion tube or blood transfusion tube. The vial needle 4 is pierced with a needle hole 5 near the piercing tip portion 2. A hollow portion 6 is formed through the main body portion 1 and the connection portion 3. The hollow portion 6 communicates with the needle hole 5. The piercing tip portion 2 constitutes a substantially curved surface of a radius of curvature of not more than 1.0 mm, desirably 1.0 to 0.1 mm, and more desirably 0.6 to 0.2 mm. In the boundary between the main body part 1 and the connection portion 3, a flange portion 7 may be formed when necessary to facilitate connection of a tube (not shown) or insertion of the piercing tip portion 2.

Fig. 2 illustrates a vial needle 14 which, similarly to the vial needle of Fig. 1, comprises a main body portion 11, a piercing tip portion 12 formed at one end of the main body portion 11, and a connection portion 13 formed at the other end of the main body portion 11. The vial needle 14 is pierced with a needle hole 15 near the piercing tip portion 12. A hollow portion 16 is formed through the main body portion 11 and the connection needle hole 15. The piercing tip portion 12 constitutes a substantially curved surface of a radius of curvature equal to the radius of curvature in the configuration of Fig. 1. Optionally, a flange portion 17 may be formed.

Fig. 3 illustrates yet another embodiment of this invention. The vial needle of this embodiment is identical in construction with that of Fig. 2. The numeral symbols of Fig. 3 which equal to those of Fig. 2 plus 10 denote identical members of configuration.

The piercing tip portions 2, 12, and 22 are invariably desired to have a tip angle, θ, in the rnage of 10° to 50°, preferably 15° to 35°.

Examples of the thermoplastic resin to be used in the medical puncture needle of this invention include polyolefins such as polypropylene and polyethylene, thermoplastic polyester resins such as polyethylene terephthalate and polybutylene terephthalate, acrylonitrile-butadiene-styrene (ABS) resin, polystyrene, acrylonitrile-styrene (AS) resin, polyamides, polycarbonates, polyacetal, polyphenylene oxide, rigid vinyl chloride resin and polyether ketone resin. In the thermoplastic resins cited above, polyolefins, ABS resin, and thermoplastic polyester resin are good selections, polypropylene and thermoplastic polyester resin are better selections, and polyethylene terephthalate is the best selection from the standpoints of resistance to chemicals, fabricability, rigidity, price, etc. Polyethylene terephthalate is desired to be of a grade having a molecular weight in the range of 15,000 to 35,000, preferably 20,000 to 30,000. Polypropylene is desired to be of a grade having a molecular weight in the range of 10,000 to 50,000, preferably 15,000 to 25,000.

The thermoplastic resin for the medical puncture needle of this invention can be selected suitably, depending on the type of sterilization which the puncture needle undergoes. When the sterilization is effected with ethylene oxide gas, for example, all the resins enumerated above are usable. Where the sterilization is effected by the use of an autoclave, polypropylene, thermoplastic polyester resin, polycarbonates, polyamides, polyacetal, polyphenylene oxide, polyether ketone resin, etc. which are resistant to heat are usable. Where the sterilization is effected with gamma ray those resins which are susceptible of deterioration by the gamma ray, among other resins cited above, are desired to incorporate therein a substance such as a hindered amine which enhances resistance to gamma ray.

The thermoplastic resin as matrix resin may incorporate therein a dispersant when necessary to ensure uniform mixture of the matrix resin with the fibrous material. When polypropylene is used as the matrix resin, for example, modified polypropylene is used as the dispersant.

Examples of the fibrous material include glass fibers (such as, for example, chopped strands), carbon fibers, rock wool, alumina fibers, and metal whiskers. In all the fibrous materials cited above, glass fibers and carbon fibers are better selections and glass fibers are the best selection from the standpoints of moldability, ease of orientation during injection, strength, price, etc. To enhance affinity of the fibrous material for the thermoplastic polyester resin, it is particularly advantageous to adopt glass fibers as the fibrous material and use a silane coupling agent for enhancement of the affinity mentioned above. The fibrous material can be used in various forms. From the standpoint of moldability of the mixture of the thermoplastic resin with the fibrous material, the individual fibers of the fibrous material are desired to have diameters not exceeding 25 µm and lengths not exceeding 20 mm. From the standpoint of strength of the mixture, they are desired to have diameters exceeding 5 µm, preferably falling in the range of 7 to 16 µm, and lengths exceeding 0.1 mm, preferably falling in the range of 0.3 to 5 mm.

The mixing ratio of the fibrous material to the thermoplastic resin is desired to be such that the amount of the fibrous material will fall in the range of 10 to 90 parts by weight, preferably 20 to 50 parts by weight, based on 100 parts by weight of the resin. If the amount of the fibrous material is less than 10 parts by weight, the produced puncture needle may fail to acquire sufficient hardness and strength. If this amount exceeds 90 parts by weight, the mixture is molded with difficulty and the produced puncture needle may suffer from prominence of fibers through the surface thereof. Optionally, the thermoplastic resin may incorporate therein pigment, oxidizing agent, neutralizing agent, seeds, and other additives.

The medical puncture needle of the present invention is produced by molding the composition prepared as described above in a prescribed shape by injection molding, transfer molding, or compression molding. From the standpoint of workability, the injection molding proves advantageous over the other methods of molding.

The hardness of the puncture needle obtained as described above is required to exceed 100 and desired to fall preferably in the range of 110 to 130, on the Rockwell hardness R scale.

Now, the present invention will be described more specifically below with reference to working examples. In the following examples, the "parts" indicated are invariably "parts by weight" unless otherwise specified.

Example 1

A medical puncture needle 4 illustrated in Fig. 1 was produced by injection molding a composition

EP 0 174 011 B1

containing 66 parts of polyethylene terephthalate (molecular weight 24,000) as a thermoplastic polyester resin, 30 parts of chopped strand glass fibers (11 μm in diameter and 0.5 mm in length) as glass fibers, and 4 parts of pigment.

The puncture needle obtained consequently was tested for physical properties by pertinent ASTM methods. The results are shown in Table 1 below.

TABLE 1

| Fundamental properties | | Methods (ASTM) |
|---|---|---|
| Specific gravity | 1.56 | D792 |
| Tensile strength (kg/cm$^2$) | 1,410 | D638 |
| Elongation (%) | 2—3 | D638 |
| Bending strength (kg/cm$^2$) | 2,050 | D790 |
| Young's modulus in flexture | 81,000 | D790 |
| Izod impact strength (kg · cm/cm-notch) | 7.0 | D256 |
| Rockwell hardness (R scale) | 120 | D785 |
| Rockwell hardness (M scale) | 100 | D785 |

The puncture needle so produced had an external diameter of 3.2 mm and the piercing tip portion of the puncture needle had a tip angle of 30° and a radius of curvature of 0.2 mm.

The puncture needle (vial needle), with silicone oil (product of Toray Silicone and marketed under trademark of SH 200, viscosity 100,000 cs) applied to the tip thereof, was tested for resistance to piercing. The objects to be pierced were a rubber plug of Fig. 4 (product of Terumo Corporation marketed under trademark designation of "Terupack") and a rubber plug of Fig. 5 (product of Otsuka Pharmaceutical Co., Ltd.) marketed under trademark designation of "Plabottle". The rubber plug of Terupack, as illustrated in Fig. 4, comprises a discharge membrane 31 applied to the leading end of a discharge tube 30, a rubber plug 32 disposed to hold down the discharge membrane 31, a discharge cap 33 adapted to secure the rubber plug 32 in place, and a dumper-proof film 34 fastened to the leading end of the discharge cap 33. The rubber plug of Plabottle, as illustrated in Fig. 5, comprises a discharge membrane 41 applied to a container body 40, a rubber plug 42 disposed to cover the discharge membrane 41, a cap 43 disposed to crown the rubber plug 42, and a caulking tube 44 adapted to tighten the cap 43.

The piercing test was carried out by using an autograph (a tension-compression tester made by Shimazu Seisakusho Ltd.) to measure the stress exerted when a given puncture needle was forced into the rubber plug at a piercing speed of 100 mm/min. The results are shown in Table 2. The numerical values of piercing resistance (in kg) shown in Table 2 represents averages of three test results obtained apiece. For comparison, the same test was performed on conventional recoat vial needles (respectively made of ABS resin and metal).

TABLE 2

| Sample | Terupack | | | Plabottle | | |
|---|---|---|---|---|---|---|
| | First piercing | Second piercing | Third piercing | First piercing | Second piercing | Third piercing |
| Example 1 | 3.7 | 3.8 | 3.8 | 2.8 | 3.4 | 3.8 |
| Conventional needle (ABS) | 6.0 | 7.7 | 7.1 | 5.0 | 5.4 | 6.0 |
| Conventional needle (metal) | 3.8 | 2.6 | 2.5 | 2.1 | 2.0 | 2.1 |

It is noted from Table 2 that the piercing resistance encountered by the puncture needle of the present invention was notably lower than that encountered by the conventional vial needle made of ABS resin and virtually equal to that encountered by the conventional metal vial needle.

4

Examples 2—3

Puncture needles were produced by following the procedure of Example 1, except that the content of glass fibers was varied as indicated below. When they were subjected to the same test, the results indicated in Table 3 were obtained.

TABLE 3

| Sample | Content of glass fibers wt. (%) | Rockwell hardness (R scale) | Terupack | | | Plabottle | | |
|--------|------|------|------|------|------|------|------|------|
| | | | First piercing | Second piercing | Third piercing | First piercing | Second piercing | Third piercing |
| Example 2 | 10 | 117 | 4.9 | 5.2 | 5.7 | 4.1 | 4.2 | 4.2 |
| Example 3 | 15 | 118 | 4.4 | 4.7 | 5.0 | 3.9 | 4.0 | 4.2 |
| Example 1 | 30 | 120 | 3.7 | 3.8 | 3.8 | 2.8 | 3.4 | 3.8 |

Example 4

Puncture needles were produced by following the procedure of Example 1, except acrylonitrile-styrene copolymer (AS resin) (product of Japan Synthetic Rubber Co., Ltd. marketed under trademark designation of Colimate S-325) and polystyrene (product of Denka K.K. marketed under trademark designation of "Denka Styrol MT-GF30") were severally used in the place of polyethylene terephthalate (PET), and the puncture needles were similarly tested. The results are shown in Table 4.

TABLE 4

| Sample | Terupack | | | Plabottle | | |
|--------|------|------|------|------|------|------|
| | First piercing | Second piercing | Third piercing | First piercing | Second piercing | Third piercing |
| AS resin | 4.5 | 4.7 | 4.9 | 3.4 | 3.8 | 4.1 |
| Polystyrene | 4.7 | 4.9 | 4.9 | 4.0 | 4.2 | 4.5 |
| Example 1 | 3.7 | 3.8 | 3.8 | 2.8 | 3.4 | 3.8 |

It is noted from Table 4 that among other resins of relatively high rigidity, polyethylene terephthalate is particularly effective in lowering the piercing resistance.

Examples 5—6

The piercing resistance is highest when the tip of the puncture needle breaks into the bulk of the rubber plug and the magnitude of this piercing resistance is variable with the shape of the piercing tip of the puncture needle.

A puncture needle was produced by following the procedure of Example 1, except that the content of glass fibers was changed to 25% by weight (Example 5), and this puncture needle was similarly tested. The results are shown in Table 5.

Separately, a puncture needle was produced by following the procedure of Example 1, except that the content of glass fibers was changed to 25% by weight and the tip of the puncture needle was sharpened instead of being rounded in a bulbous form (Example 6). This puncture needle was similarly tested. The results are shown in Table 5.

TABLE 5

| Sample | Terupack | | | Plabottle | | |
|--------|------|------|------|------|------|------|
| | First piercing | Second piercing | Third piercing | First piercing | Second piercing | Third piercing |
| Example 5 | 6.2 | 6.2 | 7.1 | 4.6 | 4.7 | 5.6 |
| Example 6 | 5.3 | 7.0 | 8.1 | 6.8 | 8.1 | 8.1 |

It is noted from Table 5 that when the tip of the puncture needle is sharpened, the piercing resistance is notably increased in the second and subsequent rounds of piercing. It is thought that such increase of the piercing resistance is caused by decreasing sharpness of the tip.

Example 7

Puncture needles were produced by following the procedure of Example 5, except that the tip angle $\theta$, of the puncture needle was varied. These puncture needles were similarly tested. The results are shown in Table 6.

TABLE 6

| | Terupack | | |
|---|---|---|---|
| Angle (°) | First piercing | Second piercing | Third piercing |
| 40 | 7.0 | 8.3 | 9.5 |
| 30 | 6.2 | 6.2 | 7.1 |
| 23 | 6.1 | 6.1 | 6.6 |
| 15 | 6.4 | 6.4 | 6.8 |

Example 8

Puncture needles produced by following the procedure of Example 1 had their tip portions coated with silicone oil (SH200) of varying viscosity and were similarly tested. The results are shown in Table 7.

TABLE 7

| | Terupack | | |
|---|---|---|---|
| Viscosity of silicone oil (cs) | First piercing | Second piercing | Third piercing |
| 100 | 5.0 | 4.7 | 4.6 |
| 1,000 | 4.8 | 5.1 | 4.9 |
| 3,000 | 4.2 | 4.3 | 4.3 |
| 30,000 | 3.8 | 4.0 | 4.1 |
| 100,000 | 3.7 | 3.8 | 3.8 |

Example 9

A medical puncture needle of Fig. 1 was produced by injection molding pellets of a composition shown in Table 8. The puncture needle had a tip angle of 50° and the tip of this puncture needle was sharp.

TABLE 8

| Material | Amount used (parts) |
|---|---|
| Matrix resin:<br>Polypropylene (homopolymer), molecular weight,<br>$M_w$=15 to 20×10⁴<br>$M_N$=2 to 4×10⁴ | 70—80 |
| Reinforcing agent:<br>Modified polypropylene | 0.5—1 |
| Antioxidant:<br>Tris(3,5-di-t-butyl-4-hydroxybenzyl) isocyanurate | 0.18—0.2 |
| Neutralizing agent:<br>Calcium stearate | 0.5—0.1 |
| Fibrous material:<br>Chopped strand glass fibers,<br>13 µm in diameter and 13 mm in length | 20—30 |

The puncture needle was similarly tested. The results are shown in Table 9.

Control 1

A puncture needle of Fig. 1 was produced by injection molding pellets of the composition of Table 8 minus the fibrous material. This puncture needle was tested by following the procedure of Example 9.

Control 2

A puncture needle was produced by following the procedure of Example 9, except that pellets of ABS resin were used instead without incorporating fibrous material. This puncture needle was tested by the procedure of Example 9. The results are shown in Table 9.

TABLE 9

| Sample | Rockwell hardness (R scale) | Plabottle | | |
|---|---|---|---|---|
| | | First piercing | Second piercing | Third piercing |
| Example 9 | 115 | 6.0 | 6.8 | 6.4 |
| Control 1 | 86 | 9.7 | impossible | impossible |
| Control 2 | 106 | 7.5 | 7.6 | 8.0 |

The numerical values of piercing resistance given above represent averages of five test results apiece. It is noted from Table 9 that the puncture needle molded with resin containing glass fibers (Example 9) was given increased hardness and the piercing tip portion of the puncture needle was prevented from yielding to pressure.

The piercing resistance was practically uniform through a plurality of rounds of piercing. The puncture needle of Control 2 represents a conventional product. It possessed strength and hardness enough to endure conditions of actual service. The puncture needle of the present invention showed piercing resistance 25% less in the first round of piercing and 20% on the average less in the first three rounds of piercing than the conventional puncture needle and, consequently, enjoyed greater ease of handling. Moreover, the puncture needle of this invention was free from the accidental extraction of needle during service, a phenomenon normally experienced in consequence of decrease of piercing resistance.

Example 10

A puncture needle illustrated in Fig. 1 was produced by injection molding pellets of the composition of Table 8 plus 0.3 part of an aromatic phosphate added as an agent for enhancing resistance to gamma ray. In this puncture needle, the tip angle was 30° and the radius of curvature was 0.2 mm. This puncture needle was tested by following the procedure of Example 9. The results are shown in Table 10. The hardness of the puncture needle was 116 as measured on the Rockwell hardness R scale.

For comparison, the puncture needle of Control 2 was similarly tested. The results are also shown in Table 10.

The numerical values of piercing resistance given in Table 10 represent averages of five test results apiece. The test for breaking strength was carried out by thrusting a given puncture needle to a depth of about 5 mm from the tip in a rubber plate about 10 mm in thickness, inclining the needle by an angle of 45 degrees, and exerting force in a vertical direction upon the base of the needle.

It is noted from Table 10 that the piercing resistance is lowered and the breaking strength is increased when the puncture needle is molded with resin containing glass fibres and the piercing tip portion of the puncture needle is formed in a substantially bulbous shape having a tip angle of 30 degrees and a radius of curvature of 0.2 mm.

It is further noted that the puncture needle of this invention, was found to enjoy an improvement of more than 13% in breaking strength. The piercing resistance was 63% in the first round of piercing and 55% on the average in the three rounds of piercing in the case of Plabottle and 16% in the first round of piercing and 27% on the average in the three rounds of piercing in the case of Terupack. Thus, the puncture needle of this invention exhibited very satisfactory results in both breaking strength and piercing resistance.

TABLE 10

| Sample | Piercing resistance (kg from rubber plug of Plabottle | | | Piercing resistance (kg) from rubber plug of Terupack | | | Breaking strength (kg) |
|---|---|---|---|---|---|---|---|
| | First | Second | Third | First | Second | Third | |
| Control 2 | 7.5 | 7.6 | 8.0 | 7.2 | 8.4 | 9.2 | 5.3 |
| Example 10 | 4.6 | 4.7 | 5.6 | 6.2 | 6.2 | 7.1 | 6.0 |

Example 11

A puncture needle illustrated in Fig. 3 was produced by injection molding pellets of the same composition as used in Example 10. In this puncture needle, the piercing tip portion 22 had a pierce forming edge portion 28 elongated more in the axial direction of the vial needle 24 than the piercing tip portion 22, specifically over a length greater than the external diameter of the main body portion 21 of the needle. The connection portion 23 of the puncture needle 24 was given a specular finish to improve tightness of contact with the tube in the fluid transfusion or blood transfusion system.

As Control 3, a puncture needle 14 illustrated in Fig. 2 was produced by injection molding pellets of ABS resin.

The test results are shown in Table 11. The numerical values of piercing resistance given in Table 11 represent averages of five test results apiece. The test for breaking strength was carried out by thrusting a given puncture needle to a depth of about 5 mm from the tip in a rubber plate about 10 mm in thickness, inclining the needle by an angle of 45 degrees, and exerting force in a vertical direction upon the base of the needle.

The object to be pierced was a diaphragm 51 used at a blood bag outlet 50 as illustrated in Fig. 6.

TABLE 11

| Sample | Piercing resistance (kg) offered by diaphragm used at the outlet | | | Breaking strength (kg) |
|---|---|---|---|---|
| | First | Second | Third | |
| Control 3 | 6.5 | 8.3 | 8.2 | 3.4 |
| Example 11 | 4.9 | 5.1 | 4.9 | 4.1 |

It is noted from Table 11 that when the puncture needle was molded with resin containing glass fibers and the needle hole forming edge portion 28 in the piercing tip portion 22 was elongated along the axial direction of the puncture needle 24, the puncture needle 24 showed lower piercing resistance and higher breaking strength than the puncture needle of Control 3.

In the puncture needle 24, the needle hole forming edge portion 28 in the piercing tip portion 22 was elongated more by 40% along the axial direction of the puncture needle 24 than the puncture needle of Control 3, the first tapered surface 29a continuing from the needle hole forming edge portion 28 was fixed at 7 degrees relative to the axial direction, the second tapered surface 29b continuing from the aforementioned first tapered surface 29a was fixed at 18 degrees relative to the axial direction, and the overall length of the puncture needle 24 from the needle hole forming edge portion 28 through the first tapered surface was consequently increased by 43% over that of the puncture needle of Control 3. Despite the resultant greater slenderness, the puncture needle of the present invention showed breaking strength 21% less on the average in the three rounds of piercing than that of the puncture needle of Control 3.

As described above, the medical puncture needle of the present invention is molded with a mixture having a fibrous material dispersed in a thermoplastic resin so as to be endowed with hardness exceeding 100 on the Rockwell hardness R scale and enabled to pierce an elastic or flexible object. Thus, it warrants safety of harding for the user, entails the phenomenon of coring least readily, enjoys enhanced breaking strength, and ensures added ease and safety of handling. Since this puncture needle can be formed by injection molding, the production of this puncture needle warrants outstanding moldability, ease of orientation during the injection molding, and enhanced strength of molded product. Further, in the puncture needle, the fibrous material is incorporated in an amount of 10 to 50 parts by weight based on 100 parts by weight of the resin. Thus, the puncture needle acquires ample hardness and satisfactory moldability. The fact that the puncture needle has a hollow portion throughout the entire length thereof except for the piercing tip portion and the piercing tip portion is in a substantially curved surface not more than 1.0 mm in radius of curvature contributes to the reduction in piercing resistance sought by this invention.

## Claims

1. A medical puncture needle formed of a hardened mixture comprising a thermoplastic resin and a fibrous material dispersed in said resin and endowed with hardness exceeding 100 on the Rockwell hardness scale R and enabled to pierce and elastic or flexible object.

2. A medical puncture needle according to Claim 1, wherein said fibrous material is incorporated in an amount of 10 to 50 parts by weight, based on 100 parts by weight of said thermoplastic resin.

3. A medical puncture needle according to Claim 1, wherein said fibrous material is glass fibers or carbon fibers.

4. A medical puncture needle according to Claim 3, wherein the individual fibers of said fibrous material have diameters not exceeding 25 μm and lengths not exceeding 30 mm.

5. A medical puncture needle according to Claim 2, wherein said thermoplastic resin is selected from the group consisting of polyolefins, ABS resin, and thermoplastic polyester resins.

6. A medical puncture needle according to Claim 5, wherein said thermoplastic resin is polyolefin.

7. A medical puncture needle according to Claim 6, wherein said polyolefin is polypropylene.

8. A medical puncture needle according to Claim 5, wherein said thermoplastic resin is a thermoplastic polyester resin.

9. A medical puncture needle according to Claim 8, wherein said thermoplastic polyester resin is polyethylene terephthalate.

10. A medical puncture needle according to Claim 2, wherein said thermoplastic resin is resistant to gamma ray.

11. A medical puncture needle according to Claim 3, wherein said fibrous material is glass fibers coated with an agent capable of enhancing affinity of said fibers for the resin.

12. A medical puncture needle according to Claim 1, having hardness exceeding 110 on the Rockwell hardness R scale.

13. A medical puncture needle according to Claim 1, having a hollow portion (6, 16, 26) throughout the entire length thereof except for a piercing tip portion (2, 12, 22) thereof and said piercing having a substantially curved surface not more than 1.0 mm in radius of curvature.

14. A medical puncture needle according to Claim 1, which is formed by injection molding.

## Patentansprüche

1. Medizinische Punktionsnadel, die aus einem gehärteten Gemisch aus einem thermoplastischen Harz und einem in diesem Harz dispergierten faserförmigen Material hergestellt ist, mit einer den Wert 100 auf der Rockwell-Härteskala R übersteigenden Härte ausgestattet ist und die Fähigkeit zum Durchstechen eines elastischen oder flexiblen Objekts besitzt.

2. Medizinische Punktionsnadel nach Anspruch 1, dadurch gekennzeichnet, daß die eingearbeitete Menge an faserförmigem Material, bezogen auf 100 Gew.-Teile des thermoplastischen Harzes, 10—50 Gew.-Teile beträgt.

3. Medizinische Punktionsnadel nach Anspruch 1, dadurch gekennzeichnet, daß das faserförmige Material aus Glasfasern oder Kohlenstoffasern besteht.

4. Medizinische Punktionsnadel nach Anspruch 3, dadurch gekennzeichnet, daß die Einzelfasern des faserförmigen Materials Durchmesser von nicht mehr als 25 μm und Längen von nicht mehr als 30 mm aufweisen.

5. Medizinische Punktionsnadel nach Anspruch 2, dadurch gekennzeichnet, daß das thermoplastische Harz aus der Gruppe Polyolefine, ABS-Harz und thermoplastische Polyesterharze ausgewählt ist.

6. Medizinische Punktionsnadel nach Anspruch 5, dadurch gekennzeichnet, daß das thermoplastische Harz aus einem Polyolefin besteht.

7. Medizinische Punktionsnadel nach Anspruch 6, dadurch gekennzeichnet, daß das Polyolefin aus Polypropylen besteht.

8. Medizinische Punktionsnadel nach Anspruch 5, dadurch gekennzeichnet, daß das thermoplastische Harz aus einem thermoplastischen Polyesterharz besteht.

9. Medizinische Punktionsnadel nach Anspruch 8, dadurch gekennzeichnet, daß das thermoplastische Polyesterharz aus Polyethylenterephthalat besteht.

10. Medizinische Punktionsnadel nach Anspruch 2, dadurch gekennzeichnet, daß das thermoplastische Harz gegen γ-Strahlung beständig ist.

11. Medizinische Punktionsnadel nach Anspruch 3, dadurch gekennzeichnet, daß das faserförmige Material aus Glasfasern besteht, die mit einem die Affinität der betreffenden Fasern zu dem Harz verstärkenden Mittel beschichtet sind.

12. Medizinische Punktionsnadel nach Anspruch 1, dadurch gekennzeichnet, daß die Härte den Wert 110 auf der Rockwell-Härteskala R übersteigt.

13. Medizinische Punktionsnadel nach Anspruch 1, dadurch gekennzeichnet, daß sie einen sich durch ihre gesamte Länge mit Ausnahme eines Durchstechspitzenteils (2, 12, 22) erstreckenden Hohlteil (6, 16, 26) aufweist und daß der Durchstechteil eine im wesentlichen gekrümmte Oberfläche eines Krümmungsradius von nicht mehr als 1,0 mm besitzt.

14. Medizinische Punktionsnadel nach Anspruch 1, dadurch gekennzeichnet, daß sie durch Spritzguß hergestellt ist.

**Revendications**

1. Une aiguille de ponction médicale formée d'un mélange durci comprenant une résine thermoplastique et une matière fibreuse dispersée dans ladite résine et douée d'une dureté de plus de 100 sur léchelle de dureté Rockwell R et capable de percer un objet élastique ou flexible.

2. Une aiguille de ponction médicale selon la revendication 1, dans laquelle ladite matière fibreuse est incorporée en quantité de 10 à 50 parties en poids pour 100 parties en poids de ladite résine thermoplastique.

3. Une aiguille de ponction médicale selon la revendication 1, dans laquelle ladite matière fibreuse consiste en fibres de verre ou en fibres de carbone.

4. Une aiguille de ponction médicale selon la revendication 3, dans laquelle les fibres individuelles de ladite matière fibreuse ont des diamètres ne dépassant pas 25 μm et des longueurs ne dépassant pas 30 mm.

5. Une aiguille de ponction médicale selon la revendication 2, dans laquelle ladite résine thermoplastique est choisie parmi les polyoléfines, la résine ABS et les résines de polyesters thermoplastiques.

6. Une aiguille de ponction médicale selon la revendication 5, dans laquelle ladite résine thermoplastique est une polyoléfine.

7. Une aiguille de ponction médicale selon la revendication 6, dans laquelle ladite polyoléfine est le polypropylène.

8. Une aiguille de ponction médicale selon la revendication 5, dans laquelle ladite résine thermoplastique est une résine de polyester thermoplastique.

9. Une aiguille de ponction médicale selon la revendication 8, dans laquelle ladite résine de polyester thermoplastique est le téréphtalate de polyéthylène.

10. Une aiguille de ponction médicale selon la revendication 2, dans laquelle ladite résine thermoplastique est résistante aux rayons gamma.

11. Une aiguille de ponction médicale selon la revendication 3, dans laquelle ladite matière fibreuse consiste en fibres de verres revêtues par un agent capable d'augmenter l'affinité desdites fibres pour la résine.

12. Une aiguille de ponction médicale selon la revendication 1, ayant une dureté de plus de 110 sur l'échelle de dureté Rockwell R.

13. Une aiguille de ponction médicale selon la revendication 1, ayant une portion creuse (6, 16, 26) sur toute sa longueur, sauf sa portion de pointe de perçage (2, 12, 22), et ladite portion de pointe de perçage ayant une surface sensiblement courbe de pas plus de 1,0 mm de rayon de courbure.

14. Une aiguille de ponction médicale selon la revendication 1 qui est formée par moulage par injection.

# FIG.1

# FIG.2

# FIG.3

# FIG.4

# FIG.5

# FIG.6